# EUROPÄISCHE PATENTSCHRIFT

(11) **EP 2 170 272 B1**
(45) Veröffentlichungstag und Bekanntmachung des Hinweises auf die Patenterteilung: **29.03.2017**
(21) Anmeldenummer: 08758803.4
(22) Anmeldetag: 28.05.2008
(51) Int. Cl.: A61K 9/00, A61K 9/14, A61K 9/16

(54) **EXTRUDATE MIT VERBESSERTER GESCHMACKSMASKIERUNG**
IMPROVED TASTE-MASKING EXTRUDATES
PRODUITS EXTRUDÉS PRÉSENTANT UN MASQUAGE AMÉLIORÉ DES GOÛTS

(30) Priorität: 08.06.2007 DE 102007026550
(43) Veröffentlichungstag der Anmeldung: 07.04.2010
(73) Patentinhaber: Bayer Intellectual Property GmbH, 40789 Monheim (DE)
(72) Erfinder: KANIKANTI, Venkata-Rangarao, 51381 Leverkusen (DE); HAMANN, Hans-Juergen, 41539 Dormagen (DE); KLEINEBUDDE, Peter, 40627 Düsseldorf (DE); MICHALK, Andrea, 65185 Wiesbaden (DE); REITZ, Claudia, CH-4053 Basel (CH)
(74) Vertreter: Gille Hrabal
(86) Internationale Anmeldenummer: PCT/EP2008/004218
(87) Internationale Veröffentlichungsnummer: WO 2008/148484

(56) Entgegenhaltungen:
- EP-A- 0 199 531
- EP-A- 0 477 979
- WO-A-96/14058
- WO-A-03/072083
- WO-A-2004/093801
- WO-A-2005/065663
- WO-A-2006/024881
- WO-A-2007/063552

## Beschreibung

Die Erfindung betrifft Extrudate enthaltend eine oder mehrere pharmazeutisch wirksame Substanzen, wobei die Extrudate einen Strangdurchmesser von 0,5 mm oder weniger aufweisen, sowie die Verwendung dieser Extrudate zur Herstellung von Arzneimitteln.

Eine kontrollierte Freigabe von Arzneistoffen hat den Vorteil für Verbraucher, schlechten Geschmack von Wirkstoffen kaschieren zu können. Das erhöht die Bereitschaft, die jeweilige Arzneiform einzunehmen, was für eine optimale Therapie wichtig ist. Dabei gibt es verschiedene Möglichkeiten in der Pharmazeutischen Technologie, Geschmack zu kaschieren. Eine Übersicht über sehr viele Methoden nebst Querverweisen zu entsprechenden Literaturstellen geben Roy [Roy, 1994] oder Sohi [Sohi et al., 2004].

Die einfachste Art der Geschmackskaschierung ist ein Zusatz von Geschmacksstoffen, wobei die Kaschierung sehr bitterer und sehr gut wasserlöslicher Stoffe problematisch sein kann. Die Vorgehensweise zum Finden der richtigen Zusätze beschreibt Bienz [Bienz, 1996].

Auch die Geschmacksmaskierung durch Verarbeitung des Wirkstoffs (Hexahydropyrazinderivate) mit einem hydrophoben Träger zu Granulaten wurde beschrieben (WO 98/03157).

Eine andere, sehr viel beschriebene Möglichkeit ist der Einsatz von Überzügen auf Arzneiformen. Neben einem Schutz vor Umwelteinflüssen kann mittels eines Überzuges die Freisetzung des Wirkstoffes aus der Arzneiform auf verschiedene Arten reguliert werden, unter anderem resultierend in einer Geschmackskaschierung. Dazu verwendete Materialien können unterschiedlichen Ursprungs und Aufbaus sein, zum Beispiel Eudragit E [Cerea et al., 2004, Lovrecich et al., 1996, Ohta und Buckton, 2004, Petereit und Weisbrod, 1999], Schellack [Pearnchob et al., 2003b, Pearnchob et al., 2003a] oder Cellulosederivate [Al-Omran et al., 2002, Li et al., 2002, Shirai et al., 1993]. Der Nachteil der Verwendung von Eudragit E ist jedoch, dass die Geschmacksmaskierung auf einer ionischen Wechselwirkung zwischen dem kationischen Hilfsstoff und anionischen Wirkstoffen beruht. Der Einsatz von Schellack ist ebenfalls nicht vorteilhaft, da es ein natürliches Polymer ist, dessen Zusammensetzung variieren kann. Abgesehen davon sind Überzüge ein weiterer Arbeitsaufwand in der Herstellung, was Zeit- und Geldinvestitionen bedingt. Allerdings beschreibt WO 2002/058669 eine feste Dispersion von Chinolon- oder Naphthyridoncarbonsäuren in einer unlöslichen Matrix, bei der es sich insbesondere um eine Schellack-Matrix handeln kann.

Der Einsatz von Ionenaustauscherharzen oder Einschlusskomplexen kann ebenfalls für eine Geschmacksmaskierung in Frage kommen. Jedoch ist im Falle der Ionenaustauscherharze eine breite Anwendbarkeit für viele Arzneistoffe nicht gegeben, da ionische Eigenschaften vorliegen müssen [Chun und Choi, 2004, Lu et al., 1991, Prompruk et al., 2005]. Einschlusskomplexe haben den Nachteil, dass nur geringe Beladungsmöglichkeiten an Wirkstoff bestehen [Sohi et al., 2004].

Fettgrundlagen finden ebenfalls Verwendung bei der Herstellung von geschmackskaschierenden Arzneiformen. Bekannt sind Untersuchungen an monolithischen Arzneiformen auf der Grundlage von Hartfett (Witepsol, Witocan) [Suzuki et al., 2003, Suzuki et al., 2004], wo zusätzlich Lecithin und Süßstoffe zur Verbesserung des Geschmacks eingesetzt werden. Der Nachteil dabei ist, dass die Fettgrundlagen aufgeschmolzen werden müssen, was wiederum zu Instabilitäten führen kann. Die ausgegossenen Tabletten mit einem Durchmesser von 2cm sind zu groß, um anhand dieser Daten Aussagen für einen Einsatz im Tierfutterbereich machen zu können. Des Weiteren wurden Vergleiche zwischen Hartfett, Glyceroldistearat und Stearinsäure als lipophile Bindemittel in der Kalt-Extrusion [Breitkreutz et al., 2003] vorgenommen, wobei auch dabei ein Einsatz von Eudragit E als Überzug nötig war, um eine Geschmackskaschierung zu erreichen. Die Extrusion von Fetten unterhalb ihres Schmelzpunktes zur Herstellung von Arzneimittelgrundstoffen wurde ebenfalls bereits beschrieben [Reitz und Kleinebudde, 2007].

EP 855 183 A2 offenbart geschmacksmaskierte Oral-Formulierungen mit Gyraseinhibitoren vom Chinolon-Typ, zu deren Herstellung der Wirkstoff mit höheren Fettsäuren und gegebenenfalls weiteren Additiven gemischt, erhitzt und nach Abkühlen granuliert oder pulverisiert wird.

Weiterhin wurden Pellets auf der Grundlage von Wachsen hergestellt [Adeyeye und Price, 1991, Adeyeye und Price, 1994, Zhou et al., 1996, Zhou et al., 1998]. Dabei wurde die Feststellung gemacht, dass die Freisetzung der Wirkstoffe von dem Schmelzpunkt des Wachses und dessen Konzentration im Pellet abhängt. Je höher der Schmelzpunkt und der Gehalt an Wachs waren, desto langsamer war die Freisetzung.

Eine weitere Möglichkeit der Geschmackskaschierung beschreiben Kim und Choi [Kim und Choi, 2004], die einen Fettkern aus Kakaobutter oder Hartfett und dem Wirkstoff herstellten und diesen mit einer Hülle aus Natriumalginat oder Carrageenan versahen. Dabei wird jedoch das Fett vollständig geschmolzen und der Herstellungsschritt des Überziehens stellt einen zusätzlichen Arbeitsschritt dar.

Weiterhin wurde Compritol® 888 ATO als matrixbildende Komponente beschrieben [Mirghani et al., 2000]. Sie beschreiben eine Herstellung von Pellets, die aus geschmolzenem Compritol®, Wirkstoff und einem Polysaccharidmantel bestehen. Die Beschichtung mit dem Polysaccharid ist wiederum ein Arbeitsschritt, der eingespart werden sollte. Li [Li et al., 2006] dagegen beschrieb den Vergleich von Matrixtabletten, die durch Verpressen am Rundläufer entweder aus einer Pulvermischung oder einer pulverisierten festen Dispersion hergestellt wurden. Die Tabletten aus der pulverisierten festen Dispersion zeigten bessere Geschmacksmaskierung. Jedoch wird für die Herstellung der festen Dispersion das Compritol® 888 ATO vollständig geschmolzen. Barthelemy [Barthelemy et al., 1999] verwendete Compritol® 888 ATO zum Überziehen von Theophyllinpellets und -granulaten. Auch hier ist das Fett komplett aufgeschmolzen worden.

Weiterhin ist der Einsatz von Phospholipiden eine Möglichkeit, Geschmack zu verbessern. Dabei wurde festgestellt, dass Phospholipide nur bitteren Geschmack maskieren andere Geschmacksrichtungen dagegen nicht beeinflussen [Katsuragi et al., 1997, Takagi et al., 2001]. Zum einen bietet sich hier also keine universelle Anwendungsmöglichkeit, da nur bitterer Geschmack kaschiert werden kann, zum anderen ist auch bekannt, dass der Zusatz von Phospholipiden die Kristallinität von Lipiden beeinflusst, was zu Instabilitäten führen kann [Schubert, 2005].

Eine weitere Studie zeigte, dass der Aufbau einer Pulvermischung ebenfalls schon zur Geschmacksmaskierung beitragen kann [Barra et al., 1999]. Die Hilfsstoffpartikel (Cellulosederivate) müssen kleiner sein als die Wirkstoffpartikel, um die Kaschierung zu ermöglichen, da die Hilfsstoffpartikel sich auf den Wirkstoffpartikeln anlagern. Der Nachteil ist in diesem Fall eine genügende Größe an Wirkstoffpartikeln, was einen Einsatz von mikronisierten Stoffen ausschließt.

WO 2003/030653 betrifft Tierfutter, in welches Wirkstoffe eingearbeitet werden und das durch Extrusion hergestellt werden kann.

WO 2003/072083 beschreibt die Schmelzextrusion einer Mischung eines basischen Arzneistoffs und eines (Meth)acrylatpolymers; die Extrudate werden anschließend zu einem Granulat oder Pulver zerkleinert. In dem erhaltenen Produkt soll eine Geschmacksisolierung des Wirkstoffs erreicht werden. WO 2004/066976 offenbart ein Verfahren zur Herstellung einer oralen Arzneiform mit unmittelbarem Zerfall durch Mischen eines anionischen Wirkstoffs, Methacrylatpolymer und einer mittel- bis langkettigen Fettsäure in der Schmelze. Nach dem Erstarren wird das Produkt gemahlen und in eine wasserlösliche Matrix eingebettet.

US 6 171 615 B1 betrifft eine Sustained-release-Formulierung des Theophyllins in einer halbfesten Matrix enthaltend polyglycolisierte Glyceride und eine Mischung von Stoffen zur Verbesserung der Kristallkeimbildung ("nucleation enhancers"). FR 2 753 904 betrifft ein Arzneimittel mit kontrollierter Freisetzung, das den Wirkstoff in einer Lipidmatrix enthält, die ihrerseits einen Behensäureester und ein hydrophobes Verdünnungsmittel umfasst.

WO 2004/014346 betrifft eine palatable Formulierung mit kontrollierter Freisetzung, die für Hobbytiere geeignet ist. Die Formulierung enthält den Wirkstoff in einer für die kontrollierte Freisetzung geeigneten kleinteiligen ("multiparticulate") Form sowie einen die Palatabilität verbessernden Zusatz.

WO 2005/097064 betrifft ein Arzneimittel, das eine Vielzahl beschichteter Teilchen enthält, deren Kern ein Matrixmaterial und ein wasserquellbares Quellmittel enthält.

Es wurde nun gefunden, dass sich Extrudate gut zur Herstellung von geschmacksmaskierten bzw. geschmackskaschierten Zubereitungen eignen, wobei insbesondere der Strangdurchmesser eine unerwartete Bedeutung hat. Üblicherweise erwartet der Fachmann bei kleineren Partikeln eine erhöhte Freisetzung der Inhaltsstoffe und damit eine schlechtere Geschmackskaschierung. Übliche pharmazeutisch verwendete Extrudate werden mit einem Strangdurchmesser in der Größenordnung von etwa 1 mm hergestellt. Nun wurde gefunden, dass bei einer Verringerung des Strangdurchmessers sich die Freisetzung der Inhaltsstoffe ebenfalls verringert, sodass Extrudate mit geringerem Strangdurchmesser zur Herstellung von geschmackskaschierten Arzneimitteln verwendet werden können.

Die Erfindung betrifft daher:
- Extrudate, enthaltend eine oder mehrere pharmazeutisch wirksame Substanzen, sowie 30 bis, 70% [m/m] Glycerolester mit C₁₂-C₁₄-Fettsäuren als Lipidgrundlage, dadurch gekennzeichet dass das Extrudat einen Strangdurchmesser von 0,5 mm oder weniger aufweist, wobei das Extrudat unterhalb seines Schmelzpunktes extrudiert wurde.
- Die Verwendung der vorstehend genannten Extrudate zur Herstellung von Arzneimitteln.

Der Strangdurchmesser der erfindungsgemäßen Extrudate beträgt höchstens 0,5 mm, bevorzugt höchstens 0,3 mm. Üblicherweise können Extrudate ab einem Durchmesser von 0,2 mm verwendet werden. Bei nichtzylindrischen Extrudaten beträgt die maximale Kantenlänge oder Ellipsenlänge höchstens 0,5 mm, bevorzugt höchstens 0,3 mm.

Die Extrudate enthalten eine für die Extrusion geeignete Grundlage aus einem thermoplastisch verformbaren Material oder einer Mischung mehrerer thermoplastisch verformbarer Materialien sowie gegebenenfalls weitere pharmazeutisch annehmbare Hilfs- und Zusatzstoffe.

Die Grundlage besteht aus thermoplastisch verformbaren Materialien wie. Bevorzugt wird eine Lipidgrundlage verwendet. Als Lipidgrundlage eignen sich z. B. Fettgrundlagen, insbesondere Glycerolester, bevorzugt handelt es sich um Ester mit C₁₂-C₂₄ Fettsäuren. Als Glycerolester seien genannt Glyceroldiester, wie z. B. Glyceroldibehenat; Glyceroltriester, wie z. B. Glyceroltrilaurat, Glyceroltrimyristat, Glyceroltripalmitat oder Glyceroltristearat, Mischungen aus Glycerolmono-, -di- und -triestern, wie z.B. Glycerolpalmitostearat. Auch genannt seien Triglyceride auf Basis von Kokosfett, Palmöl und/oder Palmkernöl (wie z. B. die unter der Bezeichnung Witocan® im Handel befindlichen Hartfette). Auch Mono- oder Diglyceride von Zitronen- und/oder Milchsäure sind einsetzbar.

Weiterhin seien genannt Wachse, insbesondere solche mit 30 bis 60 Kohlenstoffatomen, wie Cetylpalmitat. Solche Lipide sind z.B. kommerziell unter den Bezeichnungen Precirol®, Compritol® und Dynasan® erhältlich. Besonders bevorzugte Beispiele sind Glyceroldibehenat und Glyceroltrimyristat. Bevorzugt sind die Fettgrundlagen pulverförmig. Viele Lipide sind polymorph und können bei Temperatur- und Druckänderungen unter Umständen metastabile Formen bilden. Bei Lagerung können unter Umständen Umwandlungen der Modifikationen auftreten und sich stabilere Modifikationen bilden. Laut Beschreibungen in der Literatur [Reitz und Kleinebudde, 2007] ist Glyceroltrimyristat (Dynasan 114®) vergleichsweise stabil gegen solche Änderungen und eignet sich daher besonders als Lipidgrundlage für Arzneimittel.

Insbesondere die als Fettgrundlagen verwendeten Stoffe kommen oft als Mischungen z. B. von Mono-, Di- und/oder Triglyceriden in den Handel. Gegenüber diesen sind einheitliche Fettgrundlagen, die im wesentlichen nur aus einer Komponente bestehen, bevorzugt. Mit diesen Hilfsstoffen hergestellte Formulierungen zeichnen sich durch eine gute Lagerstabilität aus.

Die eingesetzte Menge an Grundlage (aus thermoplastisch verformbaren Materialien) hängt von der Menge der sonstigen Inhaltsstoffe der Extrudate ab. und werden 30 bis 70% [m/m] werden eingesetzt.

Die erfindungsgemäßen Extrudate können gegebenenfalls einen oder mehrere weitere Hilfs- und Zusatzstoffe enthalten. Als solche kommen in Frage: Fließregulierungsmittel, bevorzugt kolloidales Siliciumdioxid in einer Konzentration von 0,2% bis 2% [m/m]; Schmiermittel, bevorzugt Magnesiumstearat oder Calciumdibehenat in einer Konzentration von 0,2% bis 5% [m/m]; Tenside, bevorzugt Lecithin in einer Konzentration von 0,5% bis 10% [m/m]. Weiterhin können Antioxidantien eingesetzt werden, es eignen sich beispielsweise Butylhydroxyanisol (BHA) oder Butylhydroxytoluol (BHT), die in üblichen Mengen verwendet werden, in der Regel 0,01 bis 0,5% [m/m], bevorzugt 0,05 bis 0,2% [m/m]. Die Wirkstofffreisetzung lässt sich beispielsweise durch Zusatz von sogenannten Porenbildnern steuern. Dies sind z. B. Zucker, besonders Lactose, Polyole, insbesondere Mannit oder Polyethylenglykole, wie z.B. Macrogol 1500. Die Porenbildner werden in einer Konzentration von 5% bis 40% [m/m], bevorzugt in einer Konzentration von 5% bis 20% [m/m] eingesetzt. Eine andere Möglichkeit der Beeinflussung der Wirkstofffreisetzung stellt der Zusatz von Zerfallshilfsmitteln dar. Dazu können sogenannte Supersprengmittel wie Crospovidon, Croscarmellose-Natrium oder quervernetzte Natriumcarboxymethylstärke eingesetzt werden. Die Supersprengmittel werden in einer Konzentration von 1% bis 15% [m/m], bevorzugt in einer Konzentration von 3% bis 10% [m/m] eingesetzt. Alternativ dazu können Stoffe eingesetzt werden, die im Sauren löslich sind und/oder Kohlendioxid entwickeln wie Magnesiumcarbonat oder Calciumcarbonat. Die Kohlendioxid freisetzenden Stoffe werden in einer Konzentration von 5% bis 15% [m/m], bevorzugt in einer Konzentration von 5% bis 10% [m/m] eingesetzt.

Als pharmazeutisch wirksame Substanzen können Arzneimittelwirkstoffe eingesetzt werden und zwar insbesondere solche, deren unangenehmer Geschmack kaschiert werden soll.

Beispielhaft seien Antibiotika genannt, wie z. B. Chinolon-Antibiotika, wobei unter dieser Bezeichung auch Verbindungen verstanden werden sollen, die sich vom Naphthyridon ableiten.

Chinolone, vorzugsweise Fluorchinolone, sind unter anderem Verbindungen, wie sie in folgenden Dokumenten offenbart sind: US 4 670 444 (Bayer AG), US 4 472 405 (Riker Labs), US 4 730 000 (Abbott), US 4 861 779 (Pfizer), US 4 382 892 (Daiichi), US 4 704 459 (Toyama), als konkrete Beispiele für Chinolone seien Pipemidsäure und Nalidixinsäure genannt; als Beispiele für Fluorchinolone seien genannt: Benofloxacin, Binfloxacin, Cinoxacin, Ciprofloxacin, Danofloxacin, Difloxacin, Enoxacin, Enrofloxacin, Fleroxacin, Ibafloxacin, Levofloxacin, Lomefloxacin, Marbofloxacin, Moxifloxacin, Norfloxacin, Ofloxacin, Orbifloxacin, Pefloxacin, Temafloxacin, Tosufloxacin, Sarafloxacin, Sparfloxacin.

Eine bevorzugte Gruppe von Fluorchinolonen sind die der Formel (I) oder (II): 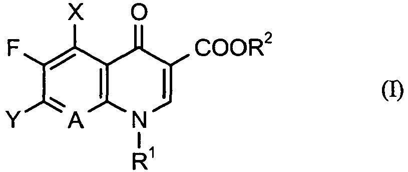 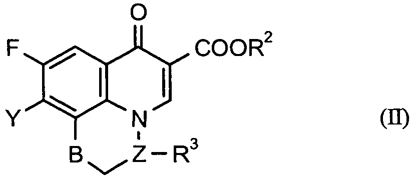 in welchen
- X: für Wasserstoff, Halogen, C₁₋₄-Alkyl, C₁₋₄-Alkoxy, NH₂ steht,
- Y: für Reste der Strukturen 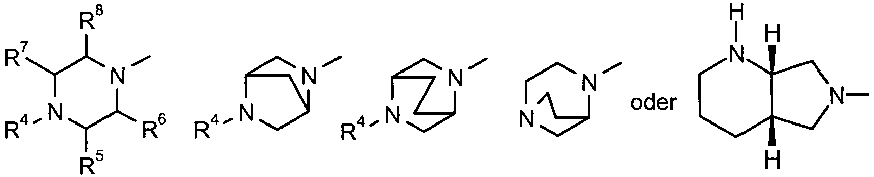
steht, worin
- R⁴: für gegebenenfalls durch Hydroxy oder Methoxy substituiertes geradkettiges oder verzweigtes C₁-C₄-Alkyl, Cyclopropyl, Acyl mit 1 bis 3 C-Atomen steht,
- R⁵: für Wasserstoff, Methyl, Phenyl, Thienyl oder Pyridyl steht,
- R⁶: für Wasserstoff oder C₁₋₄-Alkyl steht,
- R⁷: für Wasserstoff oder C₁₋₄-Alkyl steht,
- R⁸: für Wasserstoff oder C₁₋₄-Alkyl steht,
sowie
- R¹: für einen Alkylrest mit 1 bis 3 Kohlenstoffatomen, Cyclopropyl, 2-Fluorethyl, Methoxy, 4-Fluorphenyl, 2,4-Difluorphenyl oder Methylamino steht,
- R²: für Wasserstoff oder gegebenenfalls durch Methoxy oder 2-Methoxyethoxy substituiertes Alkyl mit 1 bis 6 Kohlenstoffatomen sowie Cyclohexyl, Benzyl, 2-Oxopropyl, Phenacyl, Ethoxycarbonylmethyl, Pivaloyloxymethyl steht,
- R³: für Wasserstoff, Methyl oder Ethyl steht und
- A: für Stickstoff, =CH-, =C(Halogen)-, =C(OCH₃)-, =C(CH₃)- oder =C(CN) steht,
- B: für Sauerstoff, gegebenenfalls durch Methyl oder Phenyl substituiertes =NH oder =CH₂ steht,
- Z: für =CH- oder =N- steht,
und deren pharmazeutisch verwendbaren Salze und Hydrate.

Die Verbindungen der Formeln (I) und (II) können in Form ihrer Racemate oder in enantiomeren Formen vorliegen.

Bevorzugt sind Verbindungen der Formel (I),
in welcher
- A: für =CH- oder =C-CN steht,
- R¹: für gegebenenfalls durch Halogen substituiertes C₁-C₃-Alkyl oder Cyclopropyl steht,
- R²: für Wasserstoff oder C₁₋₄-Alkyl steht,
- Y: für Reste der Strukturen 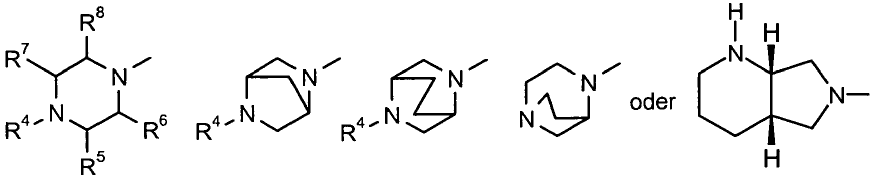
steht, worin
- R⁴: für gegebenenfalls durch Hydroxy substituiertes geradkettiges oder verzweigtes C ₁-C₃-Alkyl, Oxalkyl mit 1 bis 4 C-Atomen steht,
- R⁵: für Wasserstoff, Methyl oder Phenyl steht,
- R⁷: für Wasserstoff oder Methyl steht,
- R⁶ und R⁸: für Wasserstoff stehen
und deren pharmazeutisch verwendbaren Hydrate und Salze.

Besonders bevorzugt sind Verbindungen der Formel (I),
in welcher
- A: für =CH- oder =C-CN steht,
- R¹: für Cyclopropyl steht,
- R²: für Wasserstoff, Methyl oder Ethyl steht,
- Y: für Reste der Strukturen 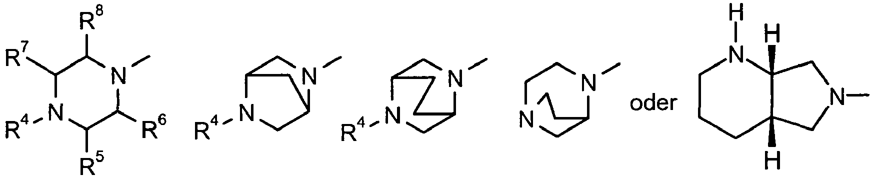
steht, worin
- R⁴: für Methyl, gegebenenfalls durch Hydroxy substituiertes Ethyl steht,
- R⁵: für Wasserstoff oder Methyl steht,
- R⁷: für Wasserstoff oder Methyl steht,
- R⁶ und R⁸: für Wasserstoff stehen
und deren pharmazeutisch verwendbaren Salze und Hydrate.

Als Salze kommen pharmazeutisch verwendbare Säureadditionssalze und basische Salze in Frage.

Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure zu verstehen. Ferner lassen sich die erfindungsgemäßen Verbindungen an saure oder basische Ionenaustauscher binden. Als pharmazeutisch verwendbare basische Salze seien die Alkalisalze, beispielsweise die Natrium- oder Kaliumsalze, die Erdalkalisalze, beispielsweise die Magnesium-, oder Calciumsalze; die Zinksalze, die Silbersalze und die Guanidiniumsalze genannt.

Unter Hydraten werden sowohl die Hydrate der Fluorchinolone selbst als auch die Hydrate von deren Salzen verstanden.

Als besonders bevorzugte Fluorchinolone seien die in WO 97/31001 beschriebenen Verbindungen genannt, insbesondere 8-Cyan-1-cyclopropyl-7-((1S,6S)-2,8-diazabicyclo[4.3.0]nonan-8-yl)-6-fluor-1,4-dihydro-4-oxo-3-chinolincarbonsäure (Pradofloxacin) mit der Formel

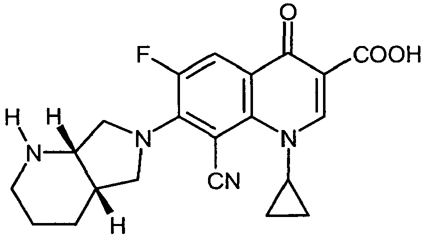

Pradofloxacin wird bevorzugt in seiner freien Form als Anhydrat, z. B. in der Modifikation B (vgl. WO 00/31076), oder als Trihydrat (vgl. WO 2005/097 789) eingesetzt.

Weiterhin besonders bevorzugt eingesetzt wird Enrofloxacin:
1-Cyclopropyl-7-(4-ethyl-1-piperazinyl)-6-fluor-1,4-dihydro-4-oxo-3-chinolincarbonsäure

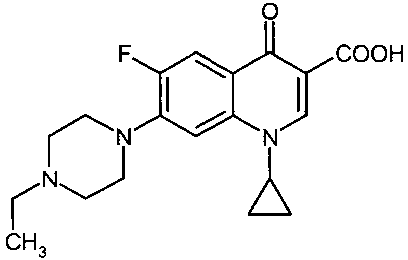

Neben Enrofloxacin und Pradofloxacin seien als bevorzugte Chinolon-Antiinfektiva noch Marbofloxacin, Orbifloxacin, Difloxacin und Ibafloxacin genannt.

Weitere geeignete Arzneimittelwirkstoffe sind z. B. Triazinone, wie beispielsweise Diclazuril, und insbesondere Ponazuril und Toltrazuril.

Weiterhin seien genannt 24-gliedrige cyclische Depsipeptide mit anthelmintischer Wirkung, z. B. PF 1022 und insbesondere Emodespide.

Auch andere Anthelmintika eignen sich. Beispielhaft seien genannt Epsiprantel und insbesondere Praziquantel.

Weiterhin als Arzneimittelwirkstoffe einsetzbar sind pharmakologisch verträgliche Phosphonsäurederivate, wobei es sich üblicherweise um organische Verbindungen handelt, die sich als Stoffwechselstimulantien und Tonika insbesondere für Nutz- und Haustiere eignen. Als bevorzugte Beispiele seien die bereits lange bekannten Verbindungen Toldimfos und insbesondere Butaphosphan (z. B. verwendet in dem Produkt Catosal®) genannt, die unter anderem der Ergänzung von Mineralstoffen (Phosphor) dienen.

Grundsätzlich eignen sich auch viele andere pharmazeutische Wirkstoffe für den Einsatz in den erfindungsgemäßen Extrudaten, da es nicht erforderlich ist, den Wirkstoff zu schmelzen. Aufgrund der geschmacksmaskierenden Wirkung der Extrudate eignen sie sich bevorzugt für unangenehm - z. B. bitter - schmeckende Wirkstoffe.

Durch die Einbettung in eine lipophile Matrix kann - je nach Natur des eingesetzten Wirkstoffs - auch eine verzögerte Freisetzung und damit ein Retard-Effekt erreicht werden.

Bei allen pharmazeutisch wirksamen Bestandteilen können - wie oben für die Chinolone ausführlicher erläutert - die entsprechenden pharmazeutisch akzeptablen Salze, Hydrate, Solvate und gegebenenfalls verschiedene Modifikationen verwendet werden.

Optisch aktive Substanzen können in Form ihrer Stereoisomere oder als Stereoisomerengemisch verwendet werden, z.B. als reine oder angereicherte Enantiomere oder als Racemate.

Die in den Extrudaten eingesetzte Menge an Wirkstoff hängt von der Wirkstärke und der angestrebten Dosierung ab. Es zeigt sich, dass auch Extrudate mit hohen Wirkstoffkonzentrationen von bis zu 80% [m/m], bevorzugt bis zu 70% [m/m], besonders bevorzugt bis zu 60% [m/m] hergestellt werden können. Übliche Konzentrationsbereiche sind z. B. 1 bis 80% [m/m], bevorzugt 5 bis 70% [m/m], besonders bevorzugt 30 bis 60% [m/m].

Die erfindungsgemäßen Extrudate werden hergestellt, indem man die Ausgangsmaterialien (die pharmazeutisch wirksame(n) Substanz(en), die Grundlage und ggf. Hilfs- und Zusatzstoffe) mischt und dann extrudiert. Die Extrusionen werden bevorzugt bei einer Temperatur durchgeführt, die nicht zu einem vollständigen Schmelzen der thermoplastisch verformbaren Materialien führt und zwar üblicherweise bei einer Temperatur im Bereich von Raumtemperatur, bevorzugt von 40° C, bis unterhalb des Schmelzbereichs der thermoplastisch verformbaren Materialien. Der Extrusionsprozess sollte bei möglichst konstanter Materialtemperatur durchgeführt werden. Für diesen Zweck eignen sich besonders beheizbare Schneckenextruder, insbesondere Zweischneckenextruder. Der extrudierte Strang hat bevorzugt einen runden Querschnitt und einen Durchmesser wie oben angegeben. Der extrudierte Strang kann direkt bei der Extrusion mit einem Messer oder in einem separaten Schritt durch schonendes Mahlen in einer üblichen Mühle, z. B. in einer Zentrifugalmühle, zerkleinert werden. Die Korngröße des erhaltenen Produkts hängt vom Durchmesser der verwendeten Düse ab, wobei die zerkleinerten Stränge maximal eine Länge aufweisen, die dem dreifachen des Strangdurchmessers entspricht. Typische Korngrößen sind z. B. 300 bis 500 µm. Gemäß einer bevorzugten Ausführungsform kann das Mahlgut noch gesiebt werden. Dadurch kann der Feinanteil entfernt werden.

Die hier gelegentlich verwendete Angabe, dass die Extrudate unterhalb ihres Schmelzpunktes extrudiert werden, ist so zu verstehen, dass die Extrudate - wie oben angegeben - bei einer Temperatur extrudiert werden, bei der die eingesetzte thermoplastische Grundlage noch nicht schmilzt. Oft haben andere Bestandteile, wie z. B. die Wirkstoffe, einen höheren Schmelzpunkt.

Bei den Extrudaten ist die Wirkstofffreisetzung bei geringerem Strangdurchmesser verringert. Solche Extrudate eignen sich also zur Geschmackskaschierung unangenehm schmeckender Inhaltsstoffe.

Die erfindungsgemäßen Extrudate können nach schonender Zerkleinerung gegebenenfalls zu geeigneten Arzneimittelformen weiter verarbeitet werden. Für die weitere Verarbeitung ist gegebenenfalls der Zusatz weiterer Hilfsstoffe erforderlich. Die erfindungsgemäß bevorzugte Arzneimittelform sind Tabletten, die ggf. an die gewünschte Anwendung angepasste Formen haben können. Andere Arzneimittelformen, die in Frage kommen sind Pasten, Suspensionen, Sachets, Kapseln etc.

Die erfindungsgemäßen Extrudate bzw. Arzneimittel eignen sich generell für die Anwendung bei Mensch und Tier. Bevorzugt werden sie in der Tierhaltung und Tierzucht bei Nutz-, Zucht-, Zoo-, Labor-, Versuchs- und Hobbytieren eingesetzt, und zwar insbesondere bei Säugetieren.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, sowie Vögel wie z.B. Hühner, Gänse, Puten, Enten, Tauben und Straußenvögel. Beispiele für bevorzugte Nutztiere sind Rind, Schaf, Schwein und Huhn.

Zu Labor- und Versuchstieren gehören Hunde, Katzen, Kaninchen und Nagetiere wie Mäuse, Ratten, Meerschweinchen und Goldhamster.

Zu den Hobbytieren gehören Hunde, Katzen, Pferde, Kaninchen, Nagetiere wie Goldhamster, Meerschweinchen, Mäuse, des Weiteren Reptilien, Amphibien und Vögel zur Heim- und Zoohaltung.

Die Anwendung der Extrudate erfolgt üblicherweise direkt oder in Form von geeigneten Zubereitungen (Arzneimittelformen) enteral, insbesondere oral.

Die enterale Anwendung der Wirkstoffe geschieht z. B. oral in Form von Granulaten, Tabletten, Kapseln, Pasten, Granulaten, Suspensionen oder medikiertem Futter.

### Geeignete Zubereitungen sind:

Feste Zubereitungen wie zum Beispiel Granulate, Pellets, Tabletten, Boli und wirkstoffhaltige Formkörper.

Zur Herstellung fester Zubereitungen werden die Wirkstoffe mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe. Geeignete Hilfsstoffe und die erforderlichen Einsatzmengen sind dem Fachmann grundsätzlich bekannt. Als Konservierungsstoff sei z. B. Sorbinsäure genannt. Als Antioxidantien eignen sich beispielsweise Butylhydroxyanisol (BHA) oder Butylhydroxytoluol (BHT). Als Farbstoffe kommen für pharmazeutische Zwecke geeignete organische und anorganische Farbstoffe bzw. Pigmente in Frage, wie z. B. Eisenoxid. Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Als weitere Hilfsmittel können Öle wie Pflanzenöle (z. B. Olivenöl, Sojaöl, Sonnenblumenöl) oder Öle tierischer Herkunft wie z. B. Fischöl eingesetzt werden. Übliche Mengen sind 0,5 bis 20% [m/m], bevorzugt 0,5 bis 10% [m/m], besonders bevorzugt 1 bis 2% [m/m].

Suspensionen können oral angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien, Lichtschutzmittel suspendiert.

Als Trägerflüssigkeiten kommen homogene Lösungsmittel oder Lösungsmittelgemische in Frage, in denen sich die jeweiligen Extrudate nicht lösen. Beispielhaft seien genannt. Physiologisch verträgliche Lösungsmittel wie Wasser, Alkohole wie Ethanol, Butanol, Glycerin, Propylenglykol, Polyethylenglykole sowie Gemische derselben.

Als Netzmittel (Dispergiermittel) können Tenside eingesetzt werden. Beispielhaft seien genannt:
nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-ß-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien z. B. genannt:
Viskositätserhöhende und die Suspension stabilisierende Stoffe wie Carboxymethylcellulose, Methylcellulose und andere Cellulose- und Stärke-Derivate, Polyacrylate, Alginate, Gelatine, Gummi arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Halbfeste Zubereitungen können oral verabreicht werden. Sie unterscheiden sich von den oben beschriebenen Suspensionen und Emulsionen nur durch ihre höhere Viskosität.

Die Wirkstoffe können auch in Kombination mit Synergisten oder mit weiteren Wirkstoffen eingesetzt werden.

### Beispiele

Soweit nicht anders angegeben handelt es sich bei den Prozentangaben um Gewichtsprozent bezogen auf die fertige Mischung.

### I. Herstellung der Extrudate

Eine Pulvermischung bestehend aus dem Wirkstoff Enrofloxacin (50% [m/m]) und den Hilfsstoffen Compritol® 888 ATO (49% [m/m]), eine Fettgrundlage mit dem Hauptbestandteil Glyceroldibehenat (es enthält auch den Mono- und Triester, sowie als kleinere Mengen an Estern mit C₁₆-C₂₀-Fettsäuren), und Aerosil® 200 (1% [m/m]), ein pyrogenes kolloidales Siliciumdioxid, dessen Einsatz zur Verbesserung der Fließfähigkeit der Pulvermasse beiträgt, wird vor der Extrusion in einem Labormischer bei Raumtemperatur gemischt (15min, 40U/min) und die Pulvermischung in den gravimetrischen Dosierer des Extruders überführt.

Für die Schmelzextrusion wird ein gleichläufiger Zweischneckenextruder mit einem Rondenwerkzeug und stumpfen Schneckenaufsätzen verwendet. Die Einstellung der Dosierrate und der Schneckengeschwindigkeit wird je nach der verwendeten Düsenplatte angepasst, um einen reproduzierbaren Prozess zu gewährleisten. Die jeweiligen Einstellungen sind in Tab. 1 aufgelistet.

**Tab. 1: Einstellungsdaten der Extrusionen**

| **Charge** | **Durchmesser** [**mm**] | **Schneckengeschwindigkeit** [**U**/**min**] | **Dosierrate [g/min**] |
|---|---|---|---|
| 1 | 0,3 | 18 | 30 |
| 2 | 0,4 | 20 | 30 |
| 3 | 0,5 | 20 | 30 |
| 4 | 1,0 | 30 | 40 |
| 5 | 2,7 | 30 | 50 |
| 6 | 5,0 | 30 | 50 |

Es werden 6 verschiedene Düsenplatten für die Herstellung der unterschiedlichen Chargen verwendet. Sie unterschieden sich hinsichtlich ihrer Düsendurchmesser, Düsenanzahl und Düsenlängen. Dabei wird darauf geachtet, bei den Düsenplatten mit Düsendurchmessern kleiner/gleich 1,0mm die offene Fläche und das Verhältnis von Länge zu Durchmesser der Düsen konstant zu halten, um stets die gleiche Beanspruchung der Extrudatmasse voraussetzen zu können. Tab. 2 zeigt die jeweiligen Parameter der einzelnen Düsenplatten.

**Tab. 2: Düsenplattenparameter**

| **Durchmesser der Düsen** [**mm**] | **5.0** | **2.7** | **1.0** | **0.5** | **0.4** | **0.3** |
|---|---|---|---|---|---|---|
| Anzahl der Düsen | 1 | 3 | 3 | 12 | 19 | 33 |
| Länge der Düsen [mm] | 5.0 | 7.5 | 2.5 | 1.25 | 1.0 | 0.75 |
| Verhältnis von Länge zu Durchmesser | 1 | 2.8 | 2.5 | 2.5 | 2.5 | 2.5 |
| Offene Fläche [mm²] | 19.64 | 17.18 | 2.36 | 2.36 | 2.39 | 2.33 |

Die Schmelzextrusionen erfolgen stets bei den gleichen Temperaturen und werden unterhalb des Schmelzbereiches von Compritol® 888 ATO (ca. 70°C) durchgeführt. Die Temperatur an der Düsenplatte war 60°C, die Zylinder des Extruders von der Düsenplatte in Richtung der Pulvereinspeisung wurden wie folgt temperiert: 60°C, 55°C, 55°C, 55°C, 55°C, 25°C, 25°C, 25°C. Nach der Schmelzextrusion werden die Extrudate mit einer Zentrifugahnühle bei 6000U/min, einem 12-Zahnrotor und einer Siebeinlage mit 1,5mm Conidurlochung gemahlen. Die Siebfraktion 315-400µm einer jeden Charge wird für alle Untersuchungen verwendet.

Weitere Rezepturen für Extrudate (soweit nicht anders angegeben handelt es sich bei den Prozentangaben um Gew.-%):

### Beispiel 2

| | |
|---|---|
| Praziquantel | 50% |
| Glycerylbehenat (Compritol® 888 ATO) | 49% |
| Kolloidales Siliciumdioxid (Aerosil® 200) | 1% |

Die drei Einsatzstoffe werden gemischt und extrudiert (Düsendurchmesser: 0.4 mm, Temperatur der Düsenplatte 60°C. Die weitere Verarbeitung der extrudierten Stränge kann wie in Beispiel 1 erfolgen.

### Beispiel 3

| | |
|---|---|
| Emodepside | 50% |
| Glycerylbehenat (Compritol® 888 ATO) | 49% |
| Kolloidales Siliciumdioxid (Aerosil® 200) | 1% |

Die drei Einsatzstoffe werden gemischt und extrudiert (Düsendurchmesser: 0.5 mm, Temperatur der Düsenplatte 60°C). Die weitere Verarbeitung der extrudierten Stränge kann wie in Beispiel 1 erfolgen.

### Beispiel 4

| | |
|---|---|
| Praziquantel | 50% |
| Glyceroltrimyristat (Dynasan 114®) | 49% |
| Kolloidales Siliciumdioxid (Aerosil® 200) | 1% |

Die drei Einsatzstoffe werden gemischt und extrudiert (Düsendurchmesser: 0.5 mm, Temperatur der Düsenplatte 50°C). Die weitere Verarbeitung der extrudierten Stränge kann wie in Beispiel 1 erfolgen.

### Beispiel 5

| | |
|---|---|
| Emodepside | 50% |
| Glyceroltrimyristat (Dynasan 114®) | 49% |
| Kolloidales Siliciumdioxid (Aerosil® 200) | 1% |

Die drei Einsatzstoffe werden gemischt und extrudiert (Düsendurchmesser: 0.4 mm, Temperatur der Düsenplatte 50°C). Die weitere Verarbeitung der extrudierten Stränge kann wie in Beispiel 1 erfolgen.

### Beispiel 6

| | |
|---|---|
| Praziquantel | 50% |
| Glyceroltrimyristat (Dynasan 114®) | 49% |
| Kolloidales Siliciumdioxid (Aerosil® 200) | 1% |

Die drei Einsatzstoffe werden gemischt und extrudiert (Düsendurchmesser: 0.33 mm, Temperatur der Düsenplatte 50°C). Die weitere Verarbeitung der extrudierten Stränge kann wie in Beispiel 1 erfolgen.

### Beispiel 7

| | |
|---|---|
| Praziquantel | 50% |
| Glyceroltripalmitat (Dynasan 116®) | 49% |
| Kolloidales Siliciumdioxid (Aerosil® 200) | 1% |

Die drei Einsatzstoffe werden gemischt und extrudiert (Düsendurchmesser: 0.33 mm, Temperatur der Düsenplatte 56°C). Die weitere Verarbeitung der extrudierten Stränge kann wie in Beispiel 1 erfolgen.

### Beispiel 8

| | |
|---|---|
| Praziquantel | 50% |
| Glyceroltristearat (Dynasan 118®) | 49% |
| Kolloidales Siliciumdioxid (Aerosil® 200) | 1 % |

Die drei Einsatzstoffe werden gemischt und extrudiert (Düsendurchmesser: 0.33 mm, Temperatur der Düsenplatte 65°C). Die weitere Verarbeitung der extrudierten Stränge kann wie in Beispiel 1 erfolgen.

### Beispiel 9

| | |
|---|---|
| Praziquantel | 50% |
| Glyceroltrimyristat (Dynasan 114®) | 49% |
| Kolloidales Siliciumdioxid (Aerosil® 200) | 1% |

Die drei Einsatzstoffe werden gemischt und extrudiert (Düsendurchmesser: 0.4 mm, Temperatur der Düsenplatte 50°C). Die weitere Verarbeitung der extrudierten Stränge kann wie in Beispiel 1 erfolgen.

### Beispiel 10

| | |
|---|---|
| Praziquantel | 50% |
| Glyceroltripalmitat (Dynasan 116®) | 49% |
| Kolloidales Siliciumdioxid (Aerosil® 200) | 1% |

Die drei Einsatzstoffe werden gemischt und extrudiert (Düsendurchmesser: 0.4 mm, Temperatur der Düsenplatte 56°C). Die weitere Verarbeitung der extrudierten Stränge kann wie in Beispiel 1 erfolgen.

### Beispiel 11

| | |
|---|---|
| Praziquantel | 50% |
| Glyceroltristearat (Dynasan 118®) | 49% |
| Kolloidales Siliciumdioxid (Aerosil® 200) | 1% |

Die drei Einsatzstoffe werden gemischt und extrudiert (Düsendurchmesser: 0.4 mm, Temperatur der Düsenplatte 65°C). Die weitere Verarbeitung der extrudierten Stränge kann wie in Beispiel 1 erfolgen.

### Beispiel 12

| | |
|---|---|
| Emodepside | 50% |
| Glyceroltripalmitat (Dynasan 116®) | 49% |
| Kolloidales Siliciumdioxid (Aerosil® 200) | 1% |

Die drei Einsatzstoffe werden gemischt und extrudiert (Düsendurchmesser: 0.33 mm, Temperatur der Düsenplatte 56°C). Die weitere Verarbeitung der extrudierten Stränge kann wie in Beispiel 1 erfolgen.

### Beispiel 13

| | |
|---|---|
| Emodepside | 50% |
| Glyceroltristearat (Dynasan 118®) | 49% |
| Kolloidales Siliciumdioxid (Aerosil® 200) | 1% |

Die drei Einsatzstoffe werden gemischt und extrudiert (Düsendurchmesser: 0.33 mm, Temperatur der Düsenplatte 65°C). Die weitere Verarbeitung der extrudierten Stränge kann wie in Beispiel 1 erfolgen.

### Beispiel 14

| | |
|---|---|
| Emodepside | 50% |
| Glyceroltrimyristat (Dynasan 114®) | 49% |
| Kolloidales Siliciumdioxid (Aerosil® 200) | 1% |

Die drei Einsatzstoffe werden gemischt und extrudiert (Düsendurchmesser: 0.33 mm, Temperatur der Düsenplatte 50°C). Die weitere Verarbeitung der extrudierten Stränge kann wie in Beispiel 1 erfolgen.

### Beispiel 15

| | |
|---|---|
| Emodepside | 50% |
| Glyceroltripalmitat (Dynasan 116®) | 49% |
| Kolloidales Siliciumdioxid (Aerosil® 200) | 1% |

Die drei Einsatzstoffe werden gemischt und extrudiert (Düsendurchmesser: 0.4 mm, Temperatur der Düsenplatte 56°C). Die weitere Verarbeitung der extrudierten Stränge kann wie in Beispiel 1 erfolgen.

### Beispiel 16

| | |
|---|---|
| Emodepside | 50% |
| Glyceroltristearat (Dynasan 118®) | 49% |
| Kolloidales Siliciumdioxid (Aerosil® 200) | 1% |

Die drei Einsatzstoffe werden gemischt und extrudiert (Düsendurchmesser: 0.4 mm, Temperatur der Düsenplatte 65°C). Die weitere Verarbeitung der extrudierten Stränge kann wie in Beispiel 1 erfolgen.

### Beispiel 17

| | |
|---|---|
| Butafosfan | 50% |
| Butylhydroxytoluol | 0.1% |
| Glyceroltrimyristat (Dynasan 114®) | 48.9% |
| Kolloidales Siliciumdioxid (Aerosil® 200) | 1% |

Die Einsatzstoffe werden gemischt und extrudiert (Düsendurchmesser: 0.4 mm, Temperatur der Düsenplatte 50°C). Die weitere Verarbeitung der extrudierten Stränge kann wie in Beispiel 1 erfolgen.

### Beispiel 18

| | |
|---|---|
| Praziquantel | 50% |
| Glyceroltrimyristat (Dynasan 114®) | 39% |
| Polyethylenglycol 1500 | 10% |
| Kolloidales Siliciumdioxid (Aerosil® 200) | 1% |

Die Einsatzstoffe werden gemischt und extrudiert (Düsendurchmesser: 0.33 mm, Temperatur der Düsenplatte 50°C). Die weitere Verarbeitung der extrudierten Stränge kann wie in Beispiel 1 erfolgen.

### Beispiel 19

| | |
|---|---|
| Emodepside | 50% |
| Glyceroltrimyristat (Dynasan 114®) | 39% |
| Polyethylenglycol 1500 | 10% |
| Kolloidales Siliciumdioxid (Aerosil® 200) | 1% |

Die Einsatzstoffe werden gemischt und extrudiert (Düsendurchmesser: 0.33 mm, Temperatur der Düsenplatte 50°C). Die weitere Verarbeitung der extrudierten Stränge kann wie in Beispiel 1 erfolgen.

### Beispiel 20

| | |
|---|---|
| Butafosfan | 50% |
| Butylhydorxytoluene | 0.1% |
| Glyceroltrimyristat (Dynasan 114®) | 38.9% |
| Polyethyleneglycol 1500 | 10% |
| Kolloidales Siliciumdioxid (Aerosil® 200) | 1% |

Die Einsatzstoffe werden gemischt und extrudiert (Düsendurchmesser: 0.33 mm, Temperatur der Düsenplatte 50°C). Die weitere Verarbeitung der extrudierten Stränge kann wie in Beispiel 1 erfolgen.

### II. Langzeituntersuchung der Arzneistofffreisetzung

Langzeituntersuchungen zur Arzneistofffreisetzung werden mittels Freisetzungsanlage nach Ph. Eur. 2.9.3, Apparatur 2 durchgeführt. Zusätzlich wird ein Sinkgefäß verwendet, in welchem sich die Probe befindet. Das Sinkgefäß liegt auf dem Boden des Freisetzungsgefäßes, der Abstand zur Unterkante des Blattrührers beträgt 2,5cm. Alle Messungen werden mit 50U/min des Blattrührers bei 37°C ± 0,5°C in 900ml Medium für 6 Proben pro Charge durchgeführt. Die Freisetzung wird bei einem pH von 7,4 (nach "Buffer Solutions" des USP27) durchgeführt unter Zusatz von 0,001% Polysorbat 20.

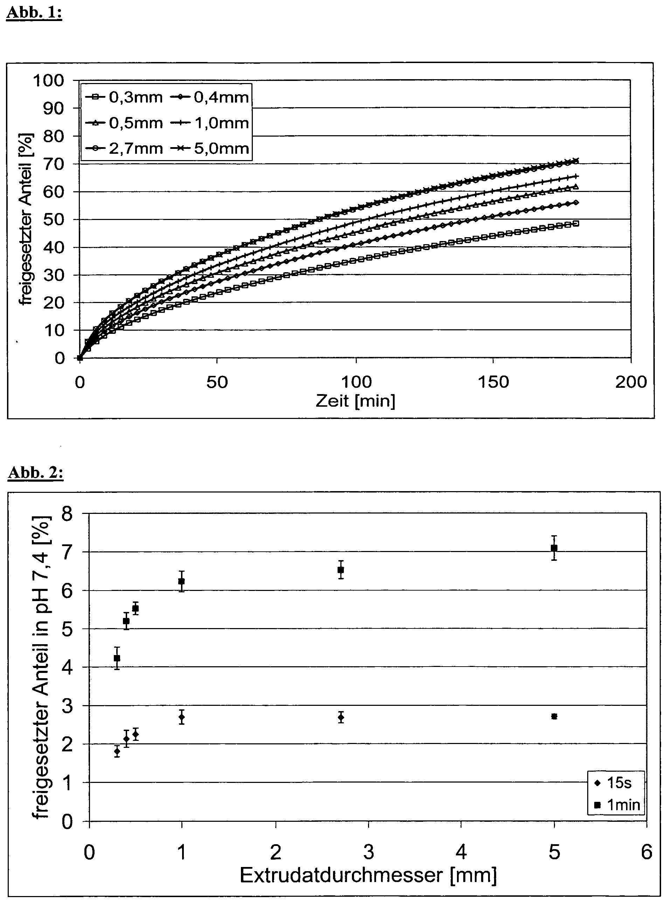

Es zeigt sich ein Unterschied in den Freigabeprofilen im Medium pH 7,4, welches dem pH-Bereich in der Mundhöhle entspricht (siehe Abb. 1: Freisetzungsprofile in pH 7,4 aller untersuchten Chargen [Mittelwerte aus 6 Bestimmungen]). Hier ist eindeutig eine stärkere Enrofloxacin-Freigabe pro Zeiteinheit bei Zunahme des originalen Durchmessers der Extrudate zu erkennen. Bei Mahlprodukten aus Extrudaten der zwei größten Originaldurchmesser gibt es keinen Unterschied im Freisetzungsprofil.

### III. Kurzzeituntersuchung der Arzneistofffreisetzung

Mit dem Verfahren der Langzeitfreisetzungsuntersuchungen können aus technischen Gründen keine Kurzzeituntersuchungen der anfänglichen Freisetzung durchgeführt werden. Für Kurzzeituntersuchungen wird daher folgendes Verfahren verwendet:
Für diese Untersuchungen wird ein Zerfallstester mit 700ml Medium pH 7,4 (wie in den Langzeituntersuchungen) bei 37°C ± 0,5°C verwendet. Die Proben werden auf drei Sinkgefäße verteilt, diese in die Probenhalterung (nach Ph. Eur. 5.5, 2.9.1. Apparatur für Test B) eingebracht und für 15s oder 1min der Test durchgeführt. Die Geschwindigkeit beim Auf- und Abfahren des Probenhalters ist konstant in allen Tests. Zum Vergleich mit den Langzeituntersuchungen werden außerdem 60min-Tests nach dem Kurzzeittest-Schema durchgeführt.

Abb. 2 zeigt die Ergebnisse aus den Kurzzeittests nach 15s und 1min (Mittelwerte ± Standardabweichungen aus 6 Proben). Es ist der freigesetzte Wirkstoffanteil gegen den Strangdurchmesser der Chargen aufgetragen. Ganz eindeutig findet eine Abnahme der Wirkstofffreigabe pro Zeiteinheit mit Abnahme des Strangdurchmessers statt. Besonders für Strangdurchmesser unterhalb von 0,5 mm ist eine deutliche Abnahme der Freisetzung zu beobachten.

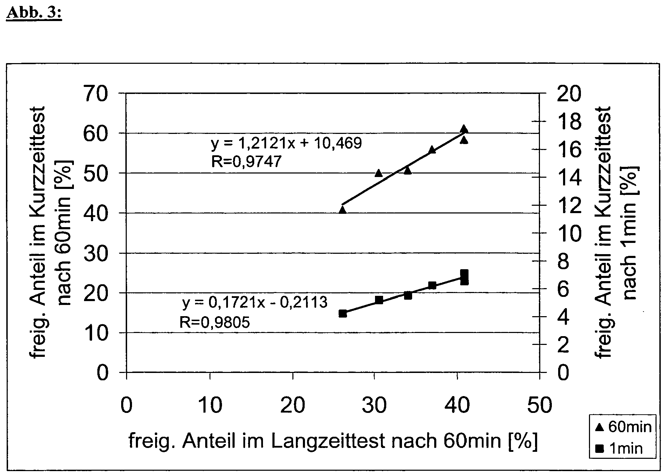

Zur Absicherung eines möglichen Vergleichs der zwei angewandten Freisetzungsuntersuchungen werden die Ergebnisse der Langzeituntersuchung mit denen der Kurzzeituntersuchung korreliert. Die 1min- und 60min-Testwerte aus dem Kurzzeittest werden jeweils den Daten der Langzeitstudie zugeordnet. Eine Korrelation der Werte ist sehr gut möglich, es besteht ein linearer Zusammenhang (siehe Abb. 3). Jeder Punkt im Diagramm entspricht einem bestimmten Durchmesser.

### LITERATURVERZEICHNIS

Adeyeye C.M., Price J.C., Development and Evaluation of Sustained-Release Ibuprofen-Wax Microspheres .1. Effect of Formulation Variables on Physical Characteristics, Pharmaceutical Research, 1991; (8): 1377-1383.
Adeyeye C.M., Price J.C., Development and Evaluation of Sustained-Release Ibuprofen-Wax Microspheres .2. In-Vitro Dissolution, Pharmaceutical Research, 1994; (11): 575-579.
Al-Omran M.F., Al-Suwayeh S.A., El-Helw A.M., Saleh S.I., Taste masking of diclofenac sodium using microencapsulation, Journal of Microencapsulation, 2002; (19): 45-52.
Barra J., Lescure F., Doelker E., Taste masking as a consequence of the organisation of powder mixes, Pharmaceutica Acta Helvetiae, 1999; (74): 37-42.
Barthelemy P., Laforet J.P., Farah N., Joachim J., Compritol (R) 888 ATO: an innovative hot-melt coating agent for prolonged-release drug formulations, European Journal of Pharmaceutics and Biopharmaceutics, 1999; (47): 87-90.
Bienz M., Taste masking strategies for drug dosage forms, Manufacturing Chemist, 1996; (67): 17-20.
Breitkreutz J., El-Saleh F., Kiera C., Kleinebudde P., Wiedey W., Pediatric drug formulations of sodium benzoate: II. Coated granules with a lipophilic binder, European Journal of Pharmaceutics and Biopharmaceutics, 2003; (56): 255-260.
Cerea M., Zheng W.J., Young C.R., McGinity J.W., A novel powder coating process for attaining taste masking and moisture protective films applied to tablets, International Journal of Pharmaceutics, 2004; (279): 127-139.
Chun M.K., Choi H.K., Preparation and characterization of enrofloxacin/carbopol complex in aqueous solution, Archives of Pharmacal Research, 2004; (27): 670-675.
Katsuragi Y., Mitsui Y., Umeda T., Otsuji K., Yamasawa S., Kurihara K., Basic studies for the practical use of bitterness inhibitors: Selective inhibition of bitterness by phospholipids, Pharmaceutical Research, 1997; (14): 720-724.
Kim E.-H., Choi H.K., Preparation of various solid-lipid beads for Drug Delivery of Enrofloxacin, Drug Delivery, 2004; (11): 365-370.
Li F.-Q., Hu J.-H., Deng J.-X., Su H., Xu S., Liu J.-Y., In vitro controlled release of sodium ferulate from Compritol 888 ATO-based matrix tablets, International Journal of Pharmaceutics, 2006; (324): 152-157.
Li S.P., Martellucci S.A., Bruce R.D., Kinyon A.C., Hay M.B., Higgins J.D., Evaluation of the film-coating properties of a hydroxyethyl cellulose/hydroxypropyl methylcellulose polymer system, Drug Development and Industrial Pharmacy, 2002; (28): 389-401.
Lovrecich M., Nobile F., Rubessa F., Zingone G., Effect of ageing on the release of indomethacin from solid dispersions with Eudragits, International Journal of Pharmaceutics, 1996; (131): 247-255.
Lu M.Y.F., Borodkin S., Woodward L., Li P., Diesner C., Hernandez L., Vadnere M., A Polymer Carrier System for Taste Masking of Macrolide Antibiotics, Pharmaceutical Research, 1991; (8): 706-712.
Mirghani A., Idkaidek N.M., Salem M.S., Najib N.M., Formulation and release behavior of diclofenac sodium in Compritol 888 matrix beads encapsulated in alginate, Drug Development and Industrial Pharmacy, 2000; (26): 791-795.
Ohta M., Buckton G., The use of inverse gas chromatography to assess the acid-base contributions to surface energies of cefditoren pivoxil and methacrylate copolymers and possible links to instability, International Journal of Pharmaceutics, 2004; (272): 121-128.
Pearnchob N., Dashevsky A., Siepmann J., Bodmeier R., Shellac used as coating material for solid pharmaceutical dosage forms: understanding the effects of formulation and processing variables, Stp Pharma Sciences, 2003a; (13): 387-396.
Pearnchob N., Siepmann J., Bodmeier R., Pharmaceutical applications of shellac: Moistureprotective and taste-masking coatings and extended-release matrix tablets, Drug Development and Industrial Pharmacy, 2003b; (29): 925-938.
Petereit H.U., Weisbrod W., Formulation and process considerations affecting the stability of solid dosage forms formulated with methacrylate copolymers, European Journal of Pharmaceutics and Biopharmaceutics, 1999; (47): 15-25.
Prompruk K., Govender T., Zhang S., Xiong C.D., Stolnik S., Synthesis of a novel PEG-block-poly(aspartic acid-stat-phenylalanine) copolymer shows potential for formation of a micellar drug carrier, International Journal of Pharmaceutics, 2005; (297): 242-253.
Reitz C, Kleinebudde P., Solid lipid extrudion of sustained release dosage forms. European Journal Of Pharmaceutics and Biopharmaceutics, 2007 doi:10.1016/j.ejpb.2007.03.008
Roy G., Taste masking in oral pharmaceuticals, Pharmaceutical technology Europe, 1994; (18): 84-99.
Schubert M.A.S.B.C., Thermal analysis of the drystallization and melting behavior of lipid matrices and lipid nanoparticles containing high amounts of lecithin, International Journal of Pharmaceutics, 2005; (298): 242-254.
Shirai Y., Sogo K., Yamamoto K., Kojima K., Fujioka H., Makita H., Nakamura Y., A Novel Fine Granule System for Masking Bitter Taste, Biological & Pharmaceutical Bulletin, 1993; (16): 172-177.
Sohi H., Sultana Y., Khar R.K., Taste masking technologies in oral pharmaceuticals: Recent developments and approaches, Drug Development and Industrial Pharmacy, 2004; (30): 429-448.
Suzuki H., Onishi H., Hisamatsu S., Masuda K., Takahashi Y., Iwata M., Machida Y., Acetaminophen-containing chewable tablets with suppressed bitterness and improved oral feeling, International Journal of Pharmaceutics, 2004; (278): 51-61.
Suzuki H., Onishi H., Takahashi Y., Iwata M., Machida Y., Development of oral acetaminophen chewable tablets with inhibited bitter taste, International Journal of Pharmaceutics, 2003; (251): 123-132.
Takagi S., Toko K., Wada K., Ohki T., Quantification of suppression of bitterness using an electronic tongue, Journal of Pharmaceutical Sciences, 2001; (90): 2042-2048.
Zhou F., Vervaet C., Remon J.P., Matrix pellets based on the combination of waxes, starches and maltodextrins, International Journal of Pharmaceutics, 1996; (133): 155-160.
Zhou F., Vervaet C., Schelkens M., Lefebvre R., Remon J.P., Bioavailability of ibuprofen from matrix pellets based on the combination of waxes and starch derivatives, International Journal of Pharmaceutics, 1998; (168): 79-84.

## Patentansprüche

1. Extrudat, enthaltend eine oder mehrere pharmazeutisch wirksame Substanz(en) sowie 30 bis 70% [m/m] Glycerolester mit C₁₂-C₂₄-Fettsäuren als Lipidgrundlage, **dadurch gekennzeichnet, dass** das Extrudat einen Strangdurchmesser von 0,5 mm oder weniger aufweist, wobei das Extrudat unterhalb seines Schmelzpunktes extrudiert wurde.

2. Extrudat gemäß Anspruch 1 mit einem Strangdurchmesser von 0,3 mm oder weniger.

3. Extrudat gemäß einem der vorstehenden Ansprüche, enthaltend Glyceroldibehenat als Lipidgrundlage.

4. Extrudat gemäß einem der Ansprüche 1 oder 2, enthaltend Glyceroltrimyristat, Glyceroltripalmitat oder Glyceroltristearat als Lipidgrundlage

5. Extrudat gemäß einem der Ansprüche 1, 2 oder 4, enthaltend Glyceroltrimyristat als Lipidgrundlage.

6. Verwendung von Extrudaten gemäß einem der vorstehenden Ansprüche zur Herstellung von Arzneimitteln.

7. Verwendung gemäß Anspruch 6 zur Herstellung von geschmackskaschierten Arzneimitteln.

8. Arzneimittel, enthaltend ein Extrudat gemäß einem der Ansprüche 1 bis 5 sowie einen oder mehrere pharmazeutisch annehmbare Hilfs- und/oder Zusatzstoffe.

## Claims

1. Extrudate comprising one or more pharmaceutically active substance(s) and 30 t0 70% [m/m] of glycerol ester with C₁₂-C₂₄ fatty acids as lipid base, **characterized in that** the extrudate has a strand diameter of 0.5 mm or less, wherein the extrudate has been extruded below its melting point.

2. Extrudate according to Claim 1 having a strand diameter of 0.3 mm or less.

3. Extrudate according to either of the preceding claims, comprising glycerol dibehenate as lipid base.

4. Extrudate according to either of Claims 1 and 2, comprising glycerol trimyristate, glycerol tripalmitate or glycerol tristearate as lipid bases.

5. Extrudate according to any of Claims 1, 2 or 4, comprising glycerol trimyristate as lipid base.

6. Use of extrudates according to any of the preceding claims for the manufacture of medicaments.

7. Use according to claim 6 for the manufacture of medicaments with concealed taste.

8. Medicament comprising an extrudate according to any of Claims 1 to 5 and one or more pharmaceutically acceptable excipients and/or additives.

## Revendications

1. Extrudat, contenant une ou plusieurs substances pharmaceutiquement actives, ainsi que 30 à 70 % [m/m] d'esters de glycérol avec des acides gras en C₁₂-C₂₄ en tant que base lipidique, **caractérisé en ce que** l'extrudat présente un diamètre de filament de 0,5 mm ou moins, l'extrudat ayant été extrudé en dessous de son point de fusion.

2. Extrudat selon la revendication 1, ayant un diamètre de filament de 0,3 mm ou moins.

3. Extrudat selon l'une quelconque des revendications précédentes, contenant du dibéhénate de glycérol en tant que base lipidique.

4. Extrudat selon l'une quelconque des revendications 1 ou 2, contenant du trimyristate de glycérol, du tripalmitate de glycérol ou du tristéarate de glycérol en tant que base lipidique.

5. Extrudat selon l'une quelconque des revendications 1, 2 ou 4, contenant du trimyristate de glycérol en tant que base lipidique.

6. Utilisation d'extrudats selon l'une quelconque des revendications précédentes pour la fabrication de médicaments.

7. Utilisation selon la revendication 6 pour la fabrication de médicaments à goût masqué.

8. Médicament, contenant un extrudat selon l'une quelconque des revendications 1 à 5, ainsi qu'un ou plusieurs adjuvants et/ou additifs pharmaceutiquement acceptables.
